# EUROPÄISCHE PATENTSCHRIFT

(11) **EP 0 809 976 B1**
(45) Veröffentlichungstag und Bekanntmachung des Hinweises auf die Patenterteilung: **21.05.2003**
(21) Anmeldenummer: 97102758.6
(22) Anmeldetag: 20.02.1997
(51) Int. Cl.: A61B 19/00, A61B 19/08, A61B 1/00, A61C 1/16

(54) **Folienschlauchabdeckung für medizinische Zwecke**
Tubular foil cover for medical use
Recouvrement en forme de film tubulaire à usages médicaux

(30) Priorität: 29.05.1996 DE 29609565 U
(43) Veröffentlichungstag der Anmeldung: 03.12.1997
(73) Patentinhaber: Udo Heisig GmbH, 85609 Aschheim-Dornach (DE)
(72) Erfinder: Heisig, Udo, 85521 Hohenbrunn-Riemerling (DE)
(74) Vertreter: Feldkamp, Rainer, Dipl.-Ing.

(56) Entgegenhaltungen:
- EP-A- 0 360 951
- DE-U- 9 418 340
- US-A- 5 325 846

## Beschreibung

Die Erfindung bezieht sich auf eine Folienschlauchabdeckung für medizinische Geräte.

Bei vielen medizinischen Geräten, beispielsweise bei Endoskopen oder Arthroskopiekameras sind diese medizinischen Geräte mit Steuer- und Auswerteeinrichtungen über langgestreckte Schläuche und/oder Kabel verbunden, die mit sterilen Abdeckungen versehen werden müssen.

Zu diesem Zweck ist es bereits bekannt, Folienschlauchabdeckungen zu verwenden, die vor dem Gebrauch mit Hilfe einer sogenannten Steckfaltung auf eine sehr kurze Länge teleskopartig zusammengeschoben oder gefaltet sind, wobei an einem Ende dieses Folienschlauches ein Haltering befestigt ist, der gleichzeitig zur Aufnahme des in seiner Länge verkürzten Folienschlauches vor Gebrauch verwendet wird.

Da die Halteringe derartiger Folienschlauchabdeckungen eine relativ geringe Breite (beispielsweise ca. 2 cm) aufweisen, kann es bei der Anwendung, d.h. wenn der Haltering über die langgestreckten Schläuche und/oder Kabel gezogen wird, um diese mit der Folienschlauchabdeckung abzudecken, dazu kommen, daß das medizinische Personal die sterile Außenseite der Schutzfolie mit den Fingern berührt, wodurch die Sterilität nicht mehr gewährleistet ist. DE-9 418 340 zeigt die Merkmale der Präambel des Anspruchs 1. US-A-5 325 846 zeigt eine Folienschlauchabdeckung wobei der Haltering ziemlich lang ist und selbst einen Griffschutz ausbildet.

Der Erfindung liegt deshalb die Aufgabe zugrunde, eine Folienschlauchabdeckung der eingangs genannten Art zu schaffen, bei deren Anwendung weitgehend ausgeschlossen werden kann, daß das medizinische Personal die sterile Außenseite der Folienschlauchabdeckung aus Versehen mit den Fingern berührt.

Diese Aufgabe wird durch die im Anspruch 1 angegebenen Merkmale gelöst.

Vorteilhafte Ausgestaltungen und Weiterbildungen der Erfindung ergeben sich aus den Unteransprüchen.

Die Erfindung wird im folgenden anhand der Zeichnungen noch näher erläutert.

In den Zeichnungen zeigen:
Figuren 1 bis 4 die Anwendung einer bekannten Folienschlauchabdeckung,
Figuren 5 bis 8 die Anwendung einer Ausführungsform der erfindungsgemäßen Folienschlauchabdeckung.

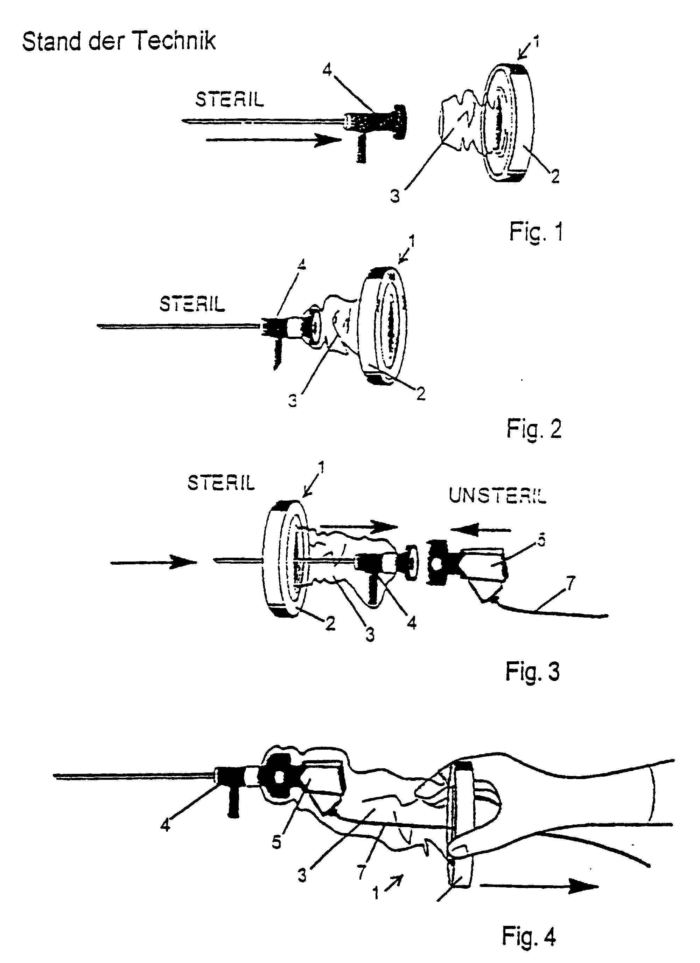

Die Figuren 1 bis 4 zeigen ein typisches Anwendungsbeispiel einer bekannten Folienschlauchabdeckung.

Um die insgesamt mit 1 bezeichnete Folienschlauchabdeckung anzuwenden, wird der sterile Teil 4 eines medizinischen Gerätes ein Stück weit durch einen Haltering 2 und ggf. eine Öffnung am Ende des Folienschlauches 3 geführt. Dabei wird das freie Ende des Folienschlauches 3 zur rechten Seite gemäß Figur 3 umgestülpt, wobei sich der steckgefaltete Folienschlauch 3 teilweise entfalten kann. Anschließend wird der unsterile Teil 5 des medizinischen Gerätes mit dem sterilen Teil 4 des medizinischen Gerätes verbunden, wobei das freie Ende des Folienschlauches dabei ggf. zwischen diesen beiden Teilen eingeklemmt wird. Nachdem die Teile 4 und 5 verbunden wurden, zieht das medizinische Personal den Haltering 2 über das langgestreckte Kabel 7, um das langgestreckte Kabel 7 mit dem Folienschlauch 3 abzudecken und damit die Sterilität zu gewährleisten. Wie dies in Figur 4 gezeigt ist, kann es dabei leicht dazu kommen, daß das medizinische Personal die sterile Außenseite des Folienschlauches 3 mit den Fingerspitzen berührt, wodurch der Folienschlauch 3 unsteril werden kann.

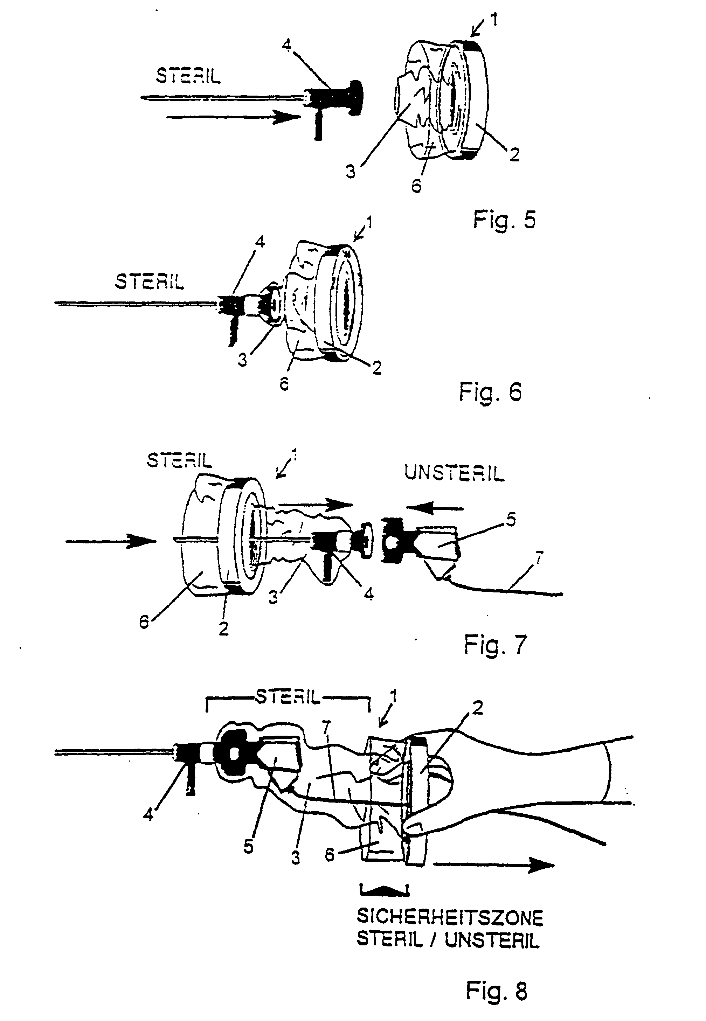

Die Figur 5 bis 8 zeigen die Anwendung einer bevorzugten Ausführungsform der erfindungsgemäßen Folienschlauchabdeckung 1.

Der Haltering 2 weist auf der dem sterilen Teil 4 des medizinischen Gerätes zugewandten Seite einen koaxial zum Folienschlauch 3 angeordneten Griffschutz 6 auf. Die Anwendung der erfindungsgemäßen Folienschlauchabdeckung entspricht im wesentlichen der Anwendung der bekannten Folienschlauchabdeckung. Wie dies insbesondere in Figur 8 deutlich gezeigt ist, wird durch den Griffschutz 6 jedoch verhindert, daß das medizinische Personal den sterilen Folienschlauch 3 versehentlich mit den Fingerspitzen berührt.

Der Griffschutz 6 kann beispielsweise einstückig mit dem Haltering 2 bzw. mit einem Teil des Halterings 2 ausgebildet sein. Diese Ausführungsform ist insbesondere dann sinnvoll, wenn der Griffschutz 6 durch ein relativ steifes Material gebildet ist.

Selbstverständlich ist es ebenfalls denkbar, den Griffschutz 6 separat aus einem geeigneten Material herzustellen, und ihn anschließend auf irgendeine geeignete Weise am Haltering 2 zu befestigen.

Weiterhin ist es denkbar, daß der Griffschutz 6 durch den Endabschnitt des Folienschlauchs 3 gebildet ist, der am Haltering 2 befestigt ist. In diesem Fall genügt es, den zur Befestigung am Haltering 2 vorgesehenen Endabschnitt des Folienschlauches 3 umzuschlagen, bevor der Folienschlauch 3 auf geeignete Weise am Haltering 2 befestigt wird. Weil der Griffschutz bei dieser Ausführungsform durch den flexiblen Folienschlauch 3 gebildet ist, ist es möglich, den Griffschutz 6 zum Transport und zur Lagerung flachzulegen bzw. ihn in den Haltering 2 einzuschlagen. Der zur Lagerung bzw. zum Transport erforderliche Raum ist dann nicht größer als bei bekannten Folienschlauchabdeckungen.

## Patentansprüche

1. Folienschlauchabdeckung (1) für medizinische Geräte (4, 5), mit einem Haltering (2), mit einem Folienschlauch (3) mit erheblicher Länge, der durch eine Faltung soweit verkürzt ist, daß er in dem Haltering (2) speicherbar ist, wobei ein Endabschnitt des Folienschlauchs (3) am Haltering (2) befestigt ist,
**dadurch gekennzeichnet, daß** sie auf der dem sterilen Teil (4) des medizinischen Gerätes zugewandten Seite einen Griffschutz (6) aufweist, der koaxial zu dem Folienschlauch (3) angeordnet ist und sich über die axiale Länge des Halteringes (2) hinaus erstreckt und daß der Griffschutz (6) aus Kunststoff und/oder Kunststoff-Folie gebildet und auf geeignete Weise am Haltering (2) befestigt ist.

2. Folienschlauchabdeckung nach Anspruch 1,
**dadurch gekennzeichnet, daß** der Griffschutz (6) durch den umgeschlagenen Endabschnitt des Folienschlauches (3) gebildet ist, der am Haltering (2) befestigt ist.

3. Folienschlauchabdeckung nach einem der vorhergehenden Ansprüche,
**dadurch gekennzeichnet, daß** der Griffschutz (6) vor Gebrauch auf eine axiale Stirnfläche des Halterings (2) flach umgelegt und/oder zumindest teilweise in den Haltering eingeschlagen ist.

## Claims

1. A tubular foil cover (1) for medical devices (4, 5) comprising a holding ring (2), a tubular foil (3) of considerable length which is shortened by folding to such an extent that it can be stored in the holding ring (2), wherein an end portion of the tubular foil (3) is fixed to the holding ring (2), **characterised in that** on the side towards the sterile part (4) of the medical device it has an anti-gripping protector (6) which is arranged coaxially with respect to the tubular foil (3) and extends beyond the axial length of the holding ring (2) and that the anti-gripping protector (6) is formed from plastic material and/or plastic foil and is suitably fixed to the holding ring (2).

2. A tubular foil cover according to claim 1 **characterised in that** the anti-gripping protector (6) is formed by the bent-over end portion of the tubular foil (3), which is fixed to the holding ring (2).

3. A tubular foil cover according to one of the preceding claims **characterised in that** prior to use the anti-gripping protector (6) is laid over flat on to an axial end face of the holding ring (2) and/or is at least partially pushed into the holding ring.

## Revendications

1. Recouvrement en forme de film tubulaire (1) pour des appareils médicaux (4, 5), avec un anneau de retenue (2), avec un film tubulaire (3) d'une longueur considérable qui est tellement raccourci par un pliage qu'il est logeable dans l'anneau de retenue (2), une section d'extrémité du film tubulaire (3) étant fixée à l'anneau de retenue (2), **caractérisé en ce qu'**il comporte une protection contre le toucher (6) sur le côté orienté vers la partie stérile (4) de l'appareil médical, disposée de manière coaxiale au film tubulaire (3) et qui s'étend plus loin que la longueur axiale de l'anneau de retenue (2) et **en ce que** la protection contre le toucher (6) est composée à partir de matière plastique et/ou de film en matière plastique et est fixée de manière appropriée à l'anneau de retenue (2).

2. Recouvrement en forme de film tubulaire selon la revendication 1, **caractérisé en ce que** la protection contre le toucher (6) est formée par la section d'extrémité rabattue du film tubulaire (3) qui est fixée à l'anneau de retenue (2).

3. Recouvrement en forme de film tubulaire selon l'une des revendications précédentes, **caractérisé en ce que** la protection contre le toucher (6) est mise à plat avant utilisation sur une face frontale axiale de l'anneau de retenue (2) et/ou est au moins en partie enfoncée dans l'anneau de retenue.
